# EUROPEAN PATENT SPECIFICATION

(11) **EP 4 194 047 B1**
(45) Date of publication and mention of the grant of the patent: **29.10.2025**
(21) Application number: 21895914.6
(22) Date of filing: 01.12.2021
(51) Int. Cl.: A61N 1/36, A61N 1/04, A61M 21/02, A61M 21/00, A61H 39/00, A61N 1/05, H04R 1/02, H04R 5/033

(54) **VAGUS NERVE STIMULATION DEVICE**
VORRICHTUNG ZUR STIMULATION DES VAGUSNERVS
DISPOSITIF DE STIMULATION DU NERF VAGUE

(30) Priority: 18.10.2021 KR 20210138039
(43) Date of publication of application: 14.06.2023
(73) Proprietor: Neurive Co., Ltd., Juchon-myeon Gimhae-si, Gyeongsangnam-do 50969 (KR)
(72) Inventor: SONG, Jae Jun, Seoul 06501 (KR); KIM, Guk Han, Anyang-si Gyeonggi-do 13961 (KR); JUNG, Yong Ho, Michuhol-gu Incheon 22100 (KR); CHOI, Hyuk, Seoul 06095 (KR); HONG, Ki Hwan, Seoul 03709 (KR)
(74) Representative: Franke, Dirk
(86) International application number: PCT/KR2021/017969
(87) International publication number: WO 2023/068442

(56) References cited:
- WO-A1-2021/051152
- CN-A- 111 603 682
- CN-A- 111 603 682
- KR-A- 20170 132 277
- US-A1- 2013 079 862
- US-A1- 2020 261 722
- US-A1- 2021 252 279
- US-A1- 2021 252 279

## Description

### Technical Field

The present disclosure relates generally to a device for vagus nerve stimulation. More particularly, the present disclosure relates to a vagus nerve stimulator that is developed so that an electrode for applying electrical stimulation to the vagus nerve located in the ear can easily make close contact with a desired position.

### Background Art

The vagus nerve is one of the cranial nerves and is also called the 10th cranial nerve. The vagus nerve runs from the brain through the face and thorax to the abdomen. It is a mixed nerve that contains parasympathetic fibers and is involved in the regulation of parasympathetic nerves acting on the heart, lungs, and digestive tract. It has the longest and most complex of the 12 pairs of cranial nerves, and contains both sensory and motor nerve fibers.

It is known that a vagus nerve stimulator for the treatment of epilepsy and depression has been approved by the US Food and Drug Administration (FDA), and it targets the cervical branch of the vagus nerve located in the neck. However, this electrical stimulation of the cervical branch of the vagus nerve is a surgical procedure involving making an incision in the skin to expose the cervical branch of the vagus nerve, winding a coil, which is an electrical body, around the cervical branch, and grafting a microchip therein. Thus, it cannot be used in the field of self-treatment by the general public.

Meanwhile, the human ear is considered as a body part where electrical stimulation can be applied to the vagus nerve by externally applying electrical stimulation to the vagus nerve because it is the main path through which the vagus nerve passes and is the region where the vagus nerve is located close to the skin surface. In oriental medicine, a method of treating diseases by ear acupuncture has been used. Since the ear is where the auricular branch of the vagus nerve is located, it can be said that the principle of ear acupuncture therapy is based on stimulation of the vagus nerve. However, the use of acupuncture is problematic in that it is not applicable to the general public.

Accordingly, a technique for applying electrical stimulation by contacting a wearer's ear with an electrode has been developed. However, there is a drawback in that the electrode is manufactured in a shape that is difficult to wear and has a low wearing comfort.

The US patent application No. US 2021/0252279 A1 discloses a transcutaneous peripheral nerve stimulation system comprising a stimulator and at least one electrode contact.

The Chinese patent application No. CN 111603682 A discloses an ear vagus nerve stimulator.

### Disclosure

### Technical Problem

Accordingly, the present disclosure has been made keeping in mind the above problems occurring in the related art, and an objective of the present disclosure is to provide a vagus nerve stimulator in which an electrode is disposed to facilitate stimulation of the vagus nerve located in the ear.

### Technical Solution

In order to achieve the above objective, according to one aspect of the present disclosure, there is provided a vagus nerve stimulator having at least two electrodes that are configured to be worn on at least one of the left and right ears and are configured to generate electrical stimulation on the vagus nerve in the auricle, the vagus nerve stimulator including: a body part configured to be located on a side of a wearer's ear; a first electrode configured to be inserted into and make contact with the external acoustic meatus of the wearer's ear; a second electrode configured to make contact with the cymba concha of the wearer's ear; a first electrode fixing part protruding from the body part and having a first electrode at an end thereof; and a second electrode fixing part protruding from the body part and having a second electrode at an end thereof. Here, when a protruding direction of the first electrode fixing part coincides with the x-axis of a three-dimensional coordinate system, an angle between a protruding direction of the second electrode fixing part and the protruding direction of the first electrode fixing part may be in a range of 10° to 20° on the xz plane and in a range of 35° to 45° on the xy plane.

The angle between the protruding direction of the second electrode fixing part and the protruding direction of the first electrode fixing part may be 14.8±2° on the xz plane and 39.7±2° on the xy plane.

An interval between a position where the first electrode fixing part protrudes from the body part and a position where the second electrode fixing part protrudes from the body part may be 14.5±2 mm.

A protruding length of the first electrode fixing part from the body part may be equal to or larger than 6 mm.

A protruding length of the second electrode fixing part from the body part may be equal to or larger than 5 mm.

The first electrode may be made of a flexible conductive polymer.

The second electrode may be made of a flexible conductive polymer.

The body part may include a forming surface from which the first electrode fixing part and the second electrode fixing part protrude, and the second electrode fixing part may be disposed at an angle of 80° to 90° with respect to a reference plane of the forming surface.

Furthermore, the body part may be configured as a pair of body parts respectively corresponding to the left and right ears. Here, the pair of body parts may be configured such that the respective second electrode fixing parts and the respective first electrode fixing parts protrude in a mirror-symmetrical shape with respect to the wearer's head.

The vagus nerve stimulator may further include a connection part connecting the pair of body parts to each other. Here, the connection part may apply a force in a direction in which the body parts make close contact with the wearer's head.

Each of the body parts may include a forming surface from which the first electrode fixing part and the second electrode fixing part protrude, and the second electrode fixing part may be disposed at an angle of 80° to 90° with respect to a reference plane of the forming surface.

### Advantageous Effects

According to the present disclosure configured as described above, by specifying the formation position of a second electrode with respect to an electrode inserted into the external acoustic meatus of the ear as a position where contact of the second electrode with the cymba concha of the ear is facilitated, it is possible to provide a vagus nerve stimulator having improved contact characteristics with the cymba concha.

In addition, by adjusting the angle of electrodes and a body part where the electrodes are formed, the electrodes are configured to make contact with the cymba concha as the body part makes close contact with the ear. Thus, it is possible to achieve close contact between the electrodes and the cymba concha by a configuration for fixing the body part.

### Description of Drawings

FIG. 1 is a schematic view of a typical human ear illustrating the contact position of an electrode in a vagus nerve stimulator.
FIG. 2 is a perspective view illustrating the configuration of a vagus nerve stimulator according to an embodiment of the present disclosure.
FIG. 3 is a partially enlarged view illustrating the configuration of the vagus nerve stimulator according to the embodiment of the present disclosure.
FIGS. 4 to 5 are schematic views illustrating the angular relationship between electrodes in the vagus nerve stimulator according to the embodiment of the present disclosure.
FIGS. 6 to 7 are views illustrating the angular relationship between a second electrode and a body part in the vagus nerve stimulator according to the embodiment of the present disclosure.

### [Description of the Reference Numerals in the Drawings]

- 100:: body part
- 110:: forming surface
- 110a:: reference plane
- 200:: first electrode
- 210:: first electrode fixing part
- 212:: conductive portion
- 300:: second electrode
- 310:: second electrode fixing part
- 312:: conductive portion
- 400:: connection part

### Mode for Invention

Reference will now be made in detail to an exemplary embodiment of the present disclosure, examples of which are illustrated in the accompanying drawings.

Various changes to the following embodiment are possible and the scope of the present disclosure is not limited to the following embodiment. In the drawings, the shape and size of elements may be exaggeratedly drawn to provide an easily understood description of the structure of the present disclosure. Wherever possible, the same reference numerals will be used throughout the drawings and the description to refer to the same or like elements or parts.

Also, when an element is referred to as being "connected" to another element, it can be "directly connected" to the other element or can be "electrically connected" to the other element with one or more intervening elements therebetween. It will be understood that terms such as "comprise", "include", and "have", when used herein, specify the presence of stated features, integers, steps, operations, elements, components, or combinations thereof, but do not preclude the presence or addition of one or more other features, integers, steps, operations, elements, components, or combinations thereof.

Further, the terms "first", "second", etc. may be used herein to describe various elements, but these elements should not be limited by these terms. These terms are only used to distinguish one element from another element. For instance, a first element discussed below could be termed a second element, and the second element could also be termed the first element.

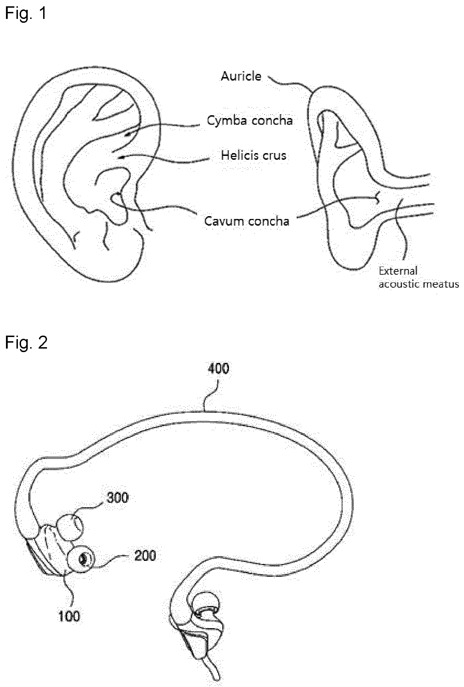

FIG. 1 is a schematic view of a typical human ear illustrating the contact position of an electrode in a vagus nerve stimulator.

The inventors of the present disclosure have developed a neuromodulation technique using the vagus nerve, unlike a transcranial magnetic stimulation method or a transcranial electrical stimulation method, in developing a neuromodulation technique for brain function activation. In particular, it is a technology that activates the limbic system and the cerebral cortex through multiple central nerve nuclei by stimulating the afferent sensory nerves non-invasively by the use of auricular vagus nerve stimulation.

In the case of transcranial magnetic stimulation or transcranial electrical stimulation, the cerebral cortex is stimulated from the outside, but in the case of the vagus nerve stimulation according to the present disclosure, it is a technology that can effectively stimulate the brainstem and the deep cerebral structures by stimulating the deep structures of the brain through the vagus nerve, which is the 10th cranial nerve.

In the human ear, the locations where the vagus nerve passes are the external acoustic meatus and the cymba concha. Here, the helicis crus, which is a cartilage structure leading from the helix, is located horizontally, and separates the cavum concha, which is the entrance to the external acoustic meatus, and the cymba concha.

In the case of the external acoustic meatus, an external acoustic meatus insertion-type structure has been developed in earphones and the like, and thus it is easy to construct an electrode making contact with the external acoustic meatus. However, due to the positional characteristics and morphological characteristics of the cymba concha, which is a concave portion lying above the helicis crus, it is not easy to construct an electrode making contact with the cymba concha.

In particular, when an electrode making contact with the external acoustic meatus and an electrode making contact with the cymba concha are provided simultaneously, there is a drawback in that the electrode making contact with the cymba concha fails to make close contact with the skin and thus fails to accurately transmit electrical stimulation. In an effort to solve this problem, the inventors of the present disclosure have researched a vagus nerve stimulator in a form that allows the position or height of an electrode making contact with the cymba concha to be adjusted so that an electrode making contact with the external acoustic meatus and the electrode making contact with the cymba concha can make close contact with the ear simultaneously, and have filed an application for patent. However, in the process including a configuration for adjusting the position of the electrode, the structure of the stimulator became complicated, resulting in an increase in manufacturing costs, and a problem of frequent failures during use.

In order to solve such problems, an objective of the present disclosure is to provide a vagus nerve stimulator capable of closely attaching a first electrode and a second electrode to the ear simultaneously by specifying the positional relationship between the first electrode making contact with the external acoustic meatus and the second electrode making contact with the cymba concha.

FIG. 2 is a perspective view illustrating the configuration of a vagus nerve stimulator according to an embodiment of the present disclosure.

The vagus nerve stimulator according to the present embodiment includes a body part 100, a first electrode 200, a second electrode 300, and a connection part 400.

The body part 100 corresponds to a body that is located on a side of a wearer's ear and is for forming the first electrode 200 and the second electrode 300 for transmitting electrical stimulation to the vagus nerve of the wearer. In this case, one body part 100 may be provided to be worn on one ear, or two body parts 100 may be provided to be worn on both ears. In the present embodiment, two body parts 100 are provided, and the first electrode 200 and the second electrode 300 are formed on each of the body parts 100.

The first electrode 200 is an electrode that is inserted into and makes contact with the external acoustic meatus of the wearer's ear through the cavum concha of the wearer's ear. Since it is inserted into and makes contact with the external acoustic meatus leading from the outside to the inside of the ear, the first electrode 200 is formed to protrude from the body part 100.

In this case, when the first electrode 200 making contact with the external acoustic meatus is made of a conductive polymer having flexibility and elastic restoring force, it may be appropriately deformed according to the size of the external acoustic meatus that is anatomically different from person to person while conducting electricity, and may exert an increased contact force against the external acoustic meatus. In addition, the electrode may be restored to its original shape and size after being removed from the external acoustic meatus, and any problem due to deformation does not occur during repeated use. As for the structure and shape of the first electrode 200 to which the conductive polymer is applied, a configuration for sealing and close contact by using a polymer in earphones and the like may be applied. In addition, the first electrode 200 to which the conductive polymer is applied may be manufactured in a detachable shape so that only the electrode is replaced, and may be manufactured in various sizes to be used interchangeably by wearers, and only the electrode may be replaced when the electrode is damaged.

Meanwhile, sound or sound waves may be transmitted through the first electrode 200 inserted into the external acoustic meatus. For example, music or sound for stabilizing purposes in a treatment process and sound waves for treatment may be transmitted. To this end, a sound unit for generating sound is provided in the body part 100, and a passage through which sound moves is formed in a space leading from the body part 100 to the first electrode 200 and in the first electrode 200. The activity of the cerebral cortex may be controlled through sound stimulation generated from the sound unit, and this effect can promote changes by influencing neural synapses related to degenerative brain diseases on the basis of neural plasticity. Non-invasive vagus nerve stimulation according to the present disclosure stimulates the cerebral cortex through the nucleus tractus solitarius, the locus coeruleus, and the nucleus basalis, thereby improving neural plasticity of the brain. Thus, it is possible to promote the formation of synapses, leading to improved learning ability. Changes in cerebral synapses caused by sound stimulation can improve the therapeutic efficacy in synergy with the improvement of neural plasticity by non-invasive vagus nerve stimulation according to the present disclosure.

The second electrode 300 is an electrode that makes contact with the cymba concha of the wearer's ear. The vagus nerve stimulator according to the present disclosure is characterized by having the electrodes that transmit electrical stimulation by respectively making contact with the external acoustic meatus and the cymba concha. The second electrode 300 transmits electrical stimulation by making contact with the cymba concha. Here, due to the concave shape of the cymba concha lying above the helicis crus, the second electrode 300 is formed to protrude from the body part 100.

In this case, when the second electrode 300 making contact with the cymba concha is made of a conductive polymer having flexibility and elastic restoring force, it may be appropriately deformed according to the structure and size of the cymba concha that is anatomically different from person to person while conducting electricity, and may exert an increased contact force against the cymba concha. In addition, almost the same effect as the case where the first electrode 200 is made of the conductive polymer can be obtained, and thus a detailed description thereof will be omitted.

Meanwhile, the first electrode 200 inserted into and making contact with the external acoustic meatus and the second electrode 300 making contact with the cymba concha, which is a concave portion lying above the helicis crus, both protrude from the body part 100. In this case, when the protruding structure and direction of the first electrode 200 and the second electrode 300 are not appropriate, they may interfere with each other and thus may fail to make close contact with the external acoustic meatus and the cymba concha. Accordingly, the present disclosure specifies the protruding structure and direction of the first electrode 200 and the second electrode 300, which will be described in more detail later.

The connection part 400 is a configuration that connects the two body parts 100 to each other. It is preferable that the connection part 400 is configured to not only connect the two body parts 100 to each other, but also exert elasticity to apply a force to the body parts 100 to make close contact with the wearer's ears.

The force of causing the body parts 100 to make close contact with the ears by the connection part 400 enables the vagus nerve stimulator according to the present embodiment to be firmly attached to the wearer, and causes the first electrode 200 and the second electrode 300 formed on the body part 100 to make close contact with the wearer's skin, thereby making it easy for electrical stimulation to be transmitted to the vagus nerve in the ear through the electrodes.

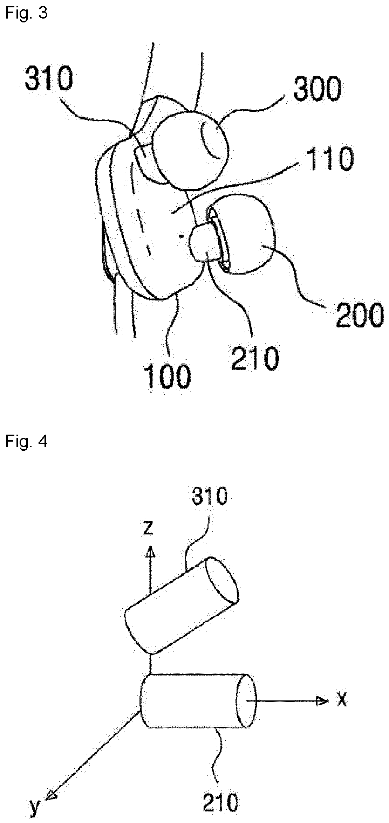

FIG. 3 is a partially enlarged view illustrating the configuration of the vagus nerve stimulator according to the embodiment of the present disclosure.

FIG. 3 illustrates an enlarged view of a body part worn on the left ear among the two body parts 100. As described above, since the first electrode 200 has to be inserted into the external acoustic meatus and the second electrode 300 has to make contact with the cymba concha, both the first electrode 200 and the second electrode 300 have to be formed to protrude from a forming surface 110 formed on each of the body parts 100 and located in a direction toward the wearer's head. Thus, the present disclosure adopts a structure in which the first electrode 200 made of the conductive polymer is attached to an end of a first electrode fixing part 210 protruding from the body part 100 and the second electrode 300 made of the conductive polymer is attached to an end of a second electrode fixing part 310 protruding from the body part 100. In this case, there is a difference in the protruding directions of the first electrode fixing part 210 and the second electrode fixing part 310 due to the positional relationship between the external acoustic meatus and the cymba concha. Thus, the protruding directions thereof have to be precisely defined so that the first electrode 200 and the second electrode 300 can make close contact with the external acoustic meatus and the cymba concha, respectively.

Since it is widely known that the external acoustic meatus of the human ear lies at an oblique angle to the front, it is preferable that the first electrode 200 inserted into and making contact with the external acoustic meatus has a forward protruding direction. Here, as the protruding direction and angle of the first electrode fixing part 210 to be inserted into the external acoustic meatus, those proposed in the technical field of earphones and the like may be employed.

On the other hand, the second electrode 300 that has to make contact with the cymba concha is not a configuration that is generally employed and has to be disposed obliquely upward with respect to the first electrode 200. For this reason, there is a problem in that the second electrode 300 may fail to make close contact with the cymba concha when it is designed incorrectly. Meanwhile, conventionally, in earphone-related technology, a structure that is rest on the cymba concha was employed for the purpose of holding earphones against ears, but this technique cannot be applied to the second electrode 300 and the second electrode fixing part 310 that transmit electrical stimulation.

As a solution for the above problem, the inventors of the present disclosure have developed a vagus nerve stimulator in which the protruding direction of the second electrode fixing part 310 is optimized on the basis of the protruding direction of the first electrode fixing part 210 to which existing techniques are applicable.

FIGS. 4 to 5 are schematic views illustrating the angular relationship between electrodes in the vagus nerve stimulator according to the embodiment of the present disclosure.

First, as illustrated in FIG. 4, in a three-dimensional coordinate system, the first electrode fixing part 210 is disposed so that the protruding direction thereof coincides with the x-axis, and the second electrode fixing part 310 is disposed so that the starting point thereof is on the z-axis. Then, the angle between a vector A with respect to the protruding direction of the first electrode fixing part 210 and a vector B with respect to the protruding direction of the second electrode fixing part 310 is specified with respect to the xy plane and the xz plane.

An angle a between the vector A with respect to the protruding direction of the first electrode fixing part 210 and the vector B with respect to the protruding direction of the second electrode fixing part 310 on the xz plane is in the range of 10° to 20°. This is an angle slightly upward when the protruding direction of the first electrode fixing part 210 is a horizontal direction. In this angular range, the second electrode 300 can make close contact with the cymba concha, which lies above the helicis crus, without being interfered with by the helicis crus. More preferably, the angle a between the vector A with respect to the protruding direction of the first electrode fixing part 210 and the vector B with respect to the protruding direction of the second electrode fixing part 310 on the xz plane is 14.8±2°.

An angle b between the vector A with respect to the protruding direction of the first electrode fixing part 210 and the vector B with respect to the protruding direction of the second electrode fixing part 310 on the xy plane is in the range of 35° to 45°. As described above, since the external acoustic meatus is disposed obliquely to the front of the face, the second electrode fixing part 310 has to be disposed so that the protruding direction thereof is oriented to the back of the face compared to the protruding direction of the first electrode fixing part 210. In this angular range, the direction in which the first electrode 200 is inserted into and makes contact with the external acoustic meatus and the direction in which the second electrode 300 makes contact with the cymba concha do not interfere with each other. More preferably, the angle b between the vector A with respect to the protruding direction of the first electrode fixing part 210 and the vector B with respect to the protruding direction of the second electrode fixing part 310 on the xy plane is 39.7±2°.

As such, when the electrodes are designed by specifying the angle between the vector A with respect to the protruding direction of the first electrode fixing part 210 and the vector B with respect to the protruding direction of the second electrode fixing part 310, it is possible to obtain the effect that the first electrode 200 is inserted into and makes close contact with the external acoustic meatus while the second electrode 200 makes close contact with the cymba concha. When being out of the above ranges, a problem occurs in that either one of the first electrode or the second electrode fails to make close contact with the ear or is difficult to wear.

In this case, as described above, due to the structural characteristics of the external acoustic meatus and the cymba concha, the first electrode fixing part 210 and the second electrode fixing part 310 have to be formed to protrude from the body part 100. The protruding length of the first electrode fixing part 210 from the body part 100 has to be equal to or larger than 6 mm, and the protruding length of the second electrode fixing part 310 from the body part 100 has to be equal to or larger than 5 mm. When the protruding length is shorter than these ranges, it becomes difficult for the electrodes to make close contact with the external acoustic meatus and the cymba concha. Meanwhile, due to the first electrode 200 made of the conductive polymer and attached to the end of the first electrode fixing part 210, the distance from the body part 100 to an end of the first electrode 200 may be larger than the protruding length of the first electrode fixing part 210, and the size of the first electrode 200 made of the conductive polymer has to be taken into consideration in the final design process. Similarly, due to the second electrode 300 made of the conductive polymer and attached to the end of the second electrode fixing part 310, the distance from the body part 100 to an end of the second electrode 300 may be larger than the protruding length of the second electrode fixing part 310, and the size of the second electrode 300 made of the conductive polymer has to be taken into consideration in the final design process. When being out of the above ranges, a problem occurs in that either one of the first electrode or the second electrode fails to make close contact with the ear or is difficult to wear.

Furthermore, on the body part 100, the first electrode fixing part 210 protrudes at a position spaced apart from the position where the second electrode fixing part 310 protrudes. Preferably, the interval between the position where the first electrode fixing part 210 protrudes and the position where the second electrode fixing part 310 protrudes is 14.5±2 mm. This is a measurement of the closest distance between the first electrode fixing part 210 and the second electrode fixing part 310, and may be appropriately adjusted depending on the width of the first electrode fixing part 210 and the second electrode fixing part 310.

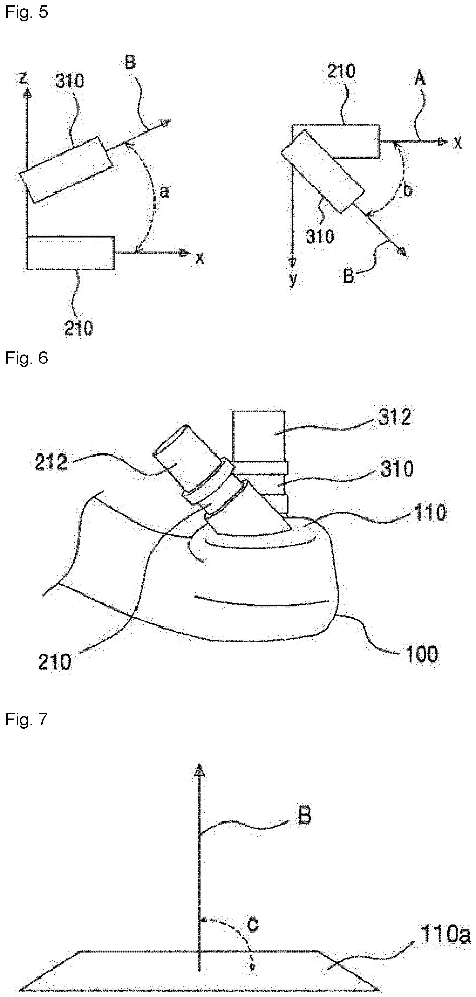

FIGS. 6 to 7 are views illustrating the angular relationship between the second electrode and the body part in the vagus nerve stimulator according to the embodiment of the present disclosure.

FIG. 6 illustrates a state in which the conductive polymer electrodes are removed from the first electrode fixing part 210 and the second electrode fixing part 310. Conductive portions 212 and 312 formed by exposing a conductive material are provided at respective portions of the first and second electrode fixing parts being in contact with the electrodes so that electricity passes through the electrodes attached to the respective ends thereof.

As described above, the vagus nerve stimulator according to the present disclosure is configure such that the first electrode 200 and the second electrode 300 are formed to protrude from the forming surface 110 of each of the body parts 100, and the opposite body parts 100 make close contact with the wearer's face (ears) by the elasticity of the connection part 400. In this case, a force is applied in a direction in which the forming surface 110 of the body part 100 makes close contact with the wearer's ears. When the protruding directions of the first electrode fixing part 210 and the second electrode fixing part 310 are appropriately configured, the force caused by the elasticity of the connection part 400 may be applied in a direction in which the first electrode 200 and the second electrode 300 make close contact with the external acoustic meatus and the cymba concha.

Here, since the forming surface 110 of the body part 100 is not a perfect plane, a reference plane 110a, which is a plane for setting an angle between the forming surface 110 and the first electrode fixing part 210 and the second electrode fixing part 310, is defined in the design process. The reference plane 110a is a plane corresponding to the forming surface 110 of the body part 100. In designing the simulator, it is preferable that a force is applied by the elasticity of the connection part 400 in a direction in which the reference plane 110a is parallel to the side of the wearer's face and makes close contact with the wearer's face (ear). Thus, as the angle between the vector B with respect to the protruding direction of the second electrode fixing part 310 and the reference plane 110a is closer to vertical, a force may be applied in the protruding direction of the second electrode fixing part 310. In view of this, when the angle between the vector B with respect to the protruding direction of the second electrode fixing part 310 and the reference plane 110a is designed to be in the range of 80° to 90°, it is possible to obtain the effect that the force caused by the elasticity of the connection part 400 is applied in a direction in which the second electrode 300 makes close contact with the cymba concha, thereby enabling close contact between the second electrode 300 and the cymba concha.

While the exemplary embodiment of the present disclosure has been described above, the embodiment is only an example of the present disclosure, and it will be understood by those skilled in the art that the present disclosure can be modified in various forms without departing from scope of the claims.

## Claims

1. A vagus nerve stimulator having at least two electrodes (200, 300) that are configured to be worn on at least one of the left and right ears and are configured to generate electrical stimulation on the vagus nerve in the auricle, the vagus nerve stimulator comprising:
a body part (100) configured to be located on a side of a wearer's ear;
a first electrode (200) configured to be inserted into and make contact with the external acoustic meatus of the wearer's ear;
a second electrode (300) configured to make contact with the cymba concha of the wearer's ear;
a first electrode fixing part (210) protruding from the body part (100) and having the first electrode at an end thereof; and
a second electrode fixing part (310) protruding from the body part (100) and having the second electrode at an end thereof,
**characterized in that** when a protruding direction of the first electrode fixing part (210) coincides with the x-axis of a three-dimensional coordinate system and the second electrode fixing part (310) is disposed so that the starting point thereof is on the z-axis, an angle between a protruding direction of the second electrode fixing part (310) and the protruding direction of the first electrode fixing part (210) is in a range of 10° to 20° on the xz plane and in a range of 35° to 45° on the xy plane.

2. The vagus nerve stimulator of claim 1, wherein the angle between the protruding direction of the second electrode fixing part (310) and the protruding direction of the first electrode fixing part is 14.8±2° on the xz plane and 39.7±2° on the xy plane.

3. The vagus nerve stimulator of claim 1, wherein an interval between a position where the first electrode fixing part (210) protrudes from the body part (100) and a position where the second electrode fixing part (310) protrudes from the body part (100) is 14.5±2 mm.

4. The vagus nerve stimulator of claim 1, wherein a protruding length of the first electrode fixing part (210) from the body part (100) is equal to or larger than 6 mm.

5. The vagus nerve stimulator of claim 1, wherein a protruding length of the second electrode fixing part (310) from the body part (100) is equal to or larger than 5 mm.

6. The vagus nerve stimulator of claim 1, wherein the first electrode is made of a flexible conductive polymer.

7. The vagus nerve stimulator of claim 1, wherein the second electrode is made of a flexible conductive polymer.

8. The vagus nerve stimulator of claim 1, wherein the body part (100) comprises a forming surface (110) from which the first electrode fixing part (210) and the second electrode fixing part (310) protrude, and
the second electrode fixing part (310) is disposed at an angle of 80° to 90° with respect to a reference plane (110a) of the forming surface (110).

9. The vagus nerve stimulator of claim 1, wherein the body part (100) is configured as a pair of body parts (100) respectively corresponding to the left and right ears,
wherein the pair of body parts (100) are configured such that the respective second electrode fixing parts and the respective first electrode fixing parts protrude in a mirror-symmetrical shape with respect to the wearer's head.

10. The vagus nerve stimulator of claim 9, further comprising a connection part (400) connecting the pair of body parts (100) to each other,
wherein the connection part (400) applies a force in a direction in which the body parts (100) make close contact with the wearer's head.

11. The vagus nerve stimulator of claim 10, wherein each of the body parts (100) comprises a forming surface (110) from which the first electrode fixing part (210) and the second electrode fixing part (310) protrude, and
the second electrode fixing part (310) is disposed at an angle of 80° to 90° with respect to a reference plane (110a) of the forming surface (110).

## Patentansprüche

1. Ein Vagusnervstimulator mit mindestens zwei Elektroden (200, 300), die so ausgelegt sind, dass sie an mindestens einem der linken und rechten Ohren getragen werden können und elektrische Stimulationen am Vagusnerv im Aurikel erzeugen, wobei der Vagusnervstimulator umfasst:
ein Körperteil (100), das so ausgelegt ist, dass es sich an einer Seite des Ohrs des Trägers befindet;
eine erste Elektrode (200), die so ausgelegt ist, dass sie in den äußeren Gehörgang des Ohrs des Trägers eingeführt wird und Kontakt mit diesem aufnimmt;
eine zweite Elektrode (300), die Kontakt mit der Cymba conchae des Ohrs des Trägers aufnimmt;
ein erstes Elektrodenbefestigungsteil (210), das aus dem Körperteil (100) hervorragt und an dessen Ende die erste Elektrode angebracht ist; und
ein zweites Elektrodenbefestigungsteil (310), das aus dem Körperteil (100) hervorragt und an dessen Ende die zweite Elektrode angebracht ist,
**dadurch gekennzeichnet, dass**, wenn die herausragende Richtung des ersten Elektrodenbefestigungsteils (210) mit der x-Achse eines dreidimensionalen Koordinatensystems übereinstimmt und das zweite Elektrodenbefestigungsteil (310) so angeordnet ist, dass dessen Anfangspunkt auf der z-Achse liegt, der Winkel zwischen der herausragenden Richtung des zweiten Elektrodenbefestigungsteils (310) und der herausragenden Richtung des ersten Elektrodenbefestigungsteils (210) im Bereich von 10° bis 20° auf der xz-Ebene und im Bereich von 35° bis 45° auf der xy-Ebene liegt.

2. Der Vagusnervstimulator nach Anspruch 1, wobei der Winkel zwischen der herausragenden Richtung des zweiten Elektrodenbefestigungsteils (310) und der des ersten Elektrodenbefestigungsteils (210) auf der xz-Ebene 14,8±2° und auf der xy-Ebene 39,7±2° beträgt.

3. Der Vagusnervstimulator nach Anspruch 1, wobei der Abstand zwischen der Position, an der das erste Elektrodenbefestigungsteil (210) aus dem Körperteil (100) hervorragt, und der Position, an der das zweite Elektrodenbefestigungsteil (310) aus dem Körperteil (100) hervorragt, 14,5±2 mm beträgt.

4. Der Vagusnervstimulator nach Anspruch 1, wobei die herausragende Länge des ersten Elektrodenbefestigungsteils (210) vom Körperteil (100) aus gleich oder größer als 6 mm ist.

5. Der Vagusnervstimulator nach Anspruch 1, wobei die herausragende Länge des zweiten Elektrodenbefestigungsteils (310) vom Körperteil (100) aus gleich oder größer als 5 mm ist.

6. Der Vagusnervstimulator nach Anspruch 1, wobei die erste Elektrode aus einem flexiblen leitfähigen Polymer besteht.

7. Der Vagusnervstimulator nach Anspruch 1, wobei die zweite Elektrode aus einem flexiblen leitfähigen Polymer besteht.

8. Der Vagusnervstimulator nach Anspruch 1, wobei das Körperteil (100) eine Formfläche (110) umfasst, von der das erste Elektrodenbefestigungsteil (210) und das zweite Elektrodenbefestigungsteil (310) hervorragen, und
das zweite Elektrodenbefestigungsteil (310) in einem Winkel von 80° bis 90° relativ zu einer Referenzfläche (110a) der Formfläche (110) angeordnet ist.

9. Der Vagusnervstimulator nach Anspruch 1, wobei das Körperteil (100) als Paar von Körperteilen (100) ausgelegt ist, die jeweils dem linken und rechten Ohr entsprechen,
wobei die beiden Körperteile (100) so ausgelegt sind, dass die jeweiligen zweiten Elektrodenbefestigungsteile und die jeweiligen ersten Elektrodenbefestigungsteile in spiegelbildlicher Form in Bezug auf den Kopf des Trägers hervorragen.

10. Der Vagusnervstimulator nach Anspruch 9, das außerdem einen Verbindungsteil (400) umfasst, der das Paar von Körperteilen (100) miteinander verbindet,
wobei der Verbindungsteil (400) eine Kraft in eine Richtung ausübt, in der die Körperteile (100) engen Kontakt mit dem Kopf des Trägers herstellen.

11. Der Vagusnervstimulator nach Anspruch 10, wobei jedes der Körperteile (100) eine Formfläche (110) umfasst, von der das erste Elektrodenbefestigungsteil (210) und das zweite Elektrodenbefestigungsteil (310) hervorragen, und
das zweite Elektrodenbefestigungsteil (310) in einem Winkel von 80° bis 90° relativ zu einer Referenzfläche (110a) der Formfläche (110) angeordnet ist.

## Revendications

1. Un stimulateur du nerf vague comportant au moins deux électrodes (200, 300) configurées pour être portées sur au moins l'une des oreilles gauche et droite et conçues pour générer une stimulation électrique du nerf vague dans l'oreille, le stimulateur du nerf vague comprenant :
un élément de corps (100) conçu pour être situé sur un côté de l'oreille de l'utilisateur ;
une première électrode (200) conçue pour être insérée dans et en contact avec le méat acoustique externe de l'oreille de l'utilisateur ;
une seconde électrode (300) conçue pour faire contact avec la conque du pavillon de l'oreille de l'utilisateur ;
une première pièce de fixation d'électrode (210) dépassant de l'élément de corps (100) et comportant la première électrode à une extrémité ; et
une seconde pièce de fixation d'électrode (310) dépassant de l'élément de corps (100) et comportant la seconde électrode à une extrémité,
**caractérisé en ce que**, lorsque la direction de protrusion de la première pièce de fixation d'électrode (210) coïncide avec l'axe x d'un système de coordonnées tridimensionnel, et que la seconde pièce de fixation d'électrode (310) est disposée de sorte que son point de départ se trouve sur l'axe z, l'angle entre la direction de protrusion de la seconde pièce de fixation d'électrode (310) et la direction de protrusion de la première pièce de fixation d'électrode (210) se trouve dans une plage de 10° à 20° sur le plan xz et dans une plage de 35° à 45° sur le plan xy.

2. Le stimulateur du nerf vague de la revendication 1, dans lequel l'angle entre la direction de protrusion de la seconde pièce de fixation d'électrode (310) et celle de la première pièce de fixation d'électrode (210) est de 14,8 ± 2° sur le plan xz et de 39,7 ± 2° sur le plan xy.

3. Le stimulateur du nerf vague de la revendication 1, dans lequel un intervalle entre la position où la première pièce de fixation d'électrode (210) dépasse de l'élément de corps (100) et la position où la seconde pièce de fixation d'électrode (310) dépasse de l'élément de corps (100) est de 14,5 ± 2 mm.

4. Le stimulateur du nerf vague de la revendication 1, dans lequel la longueur de protrusion de la première pièce de fixation d'électrode (210) par rapport à l'élément de corps (100) est supérieure ou égale à 6 mm.

5. Le stimulateur du nerf vague de la revendication 1, dans lequel la longueur de protrusion de la seconde pièce de fixation d'électrode (310) par rapport à l'élément de corps (100) est supérieure ou égale à 5 mm.

6. Le stimulateur du nerf vague de la revendication 1, dans lequel la première électrode est faite d'un polymère conducteur flexible.

7. Le stimulateur du nerf vague de la revendication 1, dans lequel la seconde électrode est faite d'un polymère conducteur flexible.

8. Le stimulateur du nerf vague de la revendication 1, dans lequel l'élément de corps (100) comprend une surface de formage (110) à partir de laquelle dépassent la première pièce de fixation d'électrode (210) et
la seconde pièce de fixation d'électrode (310), et la seconde pièce de fixation d'électrode (310) est disposée à un angle de 80° à 90° par rapport à un plan de référence (110a) de la surface de formage (110).

9. Le stimulateur du nerf vague de la revendication 1, dans lequel l'élément de corps (100) est configuré comme une paire d'éléments de corps (100) correspondant respectivement aux oreilles gauche et droite,
où la paire d'éléments de corps (100) est conçue de façon à ce que les deux pièces de fixation d'électrode respectives et les deux pièces de fixation d'électrode respectives dépassent selon une forme miroir par rapport à la tête de l'utilisateur.

10. Le stimulateur du nerf vague de la revendication 9, comprenant en outre une pièce de connexion (400) connectant la paire d'éléments de corps (100) entre eux,
où la pièce de connexion (400) exerce une force dans une direction permettant aux éléments de corps (100) de se rapprocher de la tête de l'utilisateur.

11. Le stimulateur du nerf vague de la revendication 10, dans lequel chacun des éléments de corps (100) comprend une surface de formage (110) à partir de laquelle dépassent la première pièce de fixation d'électrode (210) et la seconde pièce de fixation d'électrode (310), et
la seconde pièce de fixation d'électrode (310) est disposée à un angle de 80° à 90° par rapport à un plan de référence (110a) de la surface de formage (110).
